# EUROPÄISCHE PATENTSCHRIFT

(11) **EP 3 076 874 B1**
(45) Veröffentlichungstag und Bekanntmachung des Hinweises auf die Patenterteilung: **24.03.2021**
(21) Anmeldenummer: 14806526.1
(22) Anmeldetag: 14.11.2014
(51) Int. Cl.: A61B 6/04

(54) **TRANSFEREINRICHTUNG FÜR EINE THERAPIELIEGE**
TRANSFER DEVICE FOR A STRETCHER
SYSTÈME DE TRANSFERT POUR LIT DE TRAITEMENT

(30) Priorität: 04.12.2013 CH 20032013
(43) Veröffentlichungstag der Anmeldung: 12.10.2016
(73) Patentinhaber: Schaer Proton AG, 8416 Flaach (CH)
(72) Erfinder: SCHÄR, Hugo, 8452 Niederwil (CH)
(74) Vertreter: Gachnang, Hans Rudolf
(86) Internationale Anmeldenummer: PCT/CH2014/000164
(87) Internationale Veröffentlichungsnummer: WO 2015/081452

(56) Entgegenhaltungen:
- EP-A1- 1 238 685
- US-A1- 2007 124 858
- US-A1- 2009 217 456

## Beschreibung

Gegenstand der vorliegenden Anmeldung ist ein System umfassend eine Transfereinrichtung für eine Therapieliege gemäss Oberbegriff des Patentanspruchs 1 für die Radioonkologie.

Transfereinrichtungen für Therapieliegen werden eingesetzt, wenn Patienten zur Tumorbekämpfung bestrahlt werden müssen. Insbesondere bei der Protonen-Bestrahlung ist eine optimale Positionierung des Patienten in der Bestrahlungsmaschine von höchster Bedeutung, um den Wirkungsbereich der Protonenstrahlen exakt auf den Tumor ausrichten zu können und dort wirken zu lassen.

Um die Lage eines Tumors exakt bestimmen zu können, muss vor der Bestrahlung in einem Diagnostikzentrum mit einer Diagnostikmaschine die exakte Lage des Tumors im Körper bestimmt werden.

Da Lage und die Form des Tumors im Körper des Patienten auch durch die Art der Lagerung des Patienten beeinflusst werden, ist es sinnvoll, den Patienten bereits in den Diagnostikmaschinen in derselben Form oder Fixierung zu lagern (z. B. dem Körper anpassbare Kissen, welche vakuumiert starr sind), wie sie in der Therapiemaschine notwendig ist. Idealerweise werden in der Diagnostik dieselben Formen respektive Lagerungsmittel für den Patienten eingesetzt, welche auch in der Therapie angewendet werden. Daraus ergibt sich der Wunsch, auch dieselben Liegen, welche die Formen aufnehmen, sowohl in der Diagnostik als auch für die Therapie zu verwenden. In der Fachsprache nennt man diese transportierbare Liege "Movable Couch".

Somit können diese Liegen auch in separaten Vorbereitungsräumen bereits eingesetzt werden. D. h., der Patient kann in der entsprechenden Form auf der Liege ausserhalb der Diagnostik respektive der Therapie stressfrei vorbereitet werden, was in der US2009217456A1 gezeigt ist, und die Verweilzeit des Patienten in den Diagnostik- als auch in den Therapieräumen an den entsprechenden Maschinen wird dadurch wesentlich verkürzt.

An den Therapiemaschinen werden die Patienten von oben und seitlich bestrahlt. Zusätzlich wird in einem beschränkten Bereich der Liege auch eine Bestrahlung von der unteren Seite durch die Couch hindurch gefordert, d. h. in diesem Bereich sind Bestrahlungen 360° um den Patienten herum möglich. Diese Zone ist ca. 1 - 1.3 m lang. Die Liege ist in diesem Bereich so zu gestalten, dass die Bestrahlung durch die Liege hindurch mit ihrer Wirkung auf den Tumor berechenbar bleibt. D. h., es können keine Kupplungsteile, Schrauben usw. angebracht werden, welche eine stabile und präzise Kopplung auf den Patientenpositioniersystemen an den Therapiemaschinen erlauben oder eine Positionierung auf den Patiententischen an den Diagnostikmaschinen ermöglichen. Die Diagnostikmaschinen haben eigene Patientenpositioniersysteme, welche einen integralen Bestandteil des zertifizierten Produktes z. B. CT, MRI oder CT-PET darstellen. Die Couches sollten idealerweise distanzfrei auf die Patientenpositioniersysteme an den Diagnostikmaschinen aufgelegt werden können.

Dieses Ziel verfolgte man bisher mit sogenannten Sliding Couches, welche von einem Transportwagen auf die Diagnostik- respektive Therapiemaschinen geschoben werden oder an der Therapiemaschine auf der Unterseite der Couch angekoppelt werden.

Ein Merkmal dieser Lösungen ist, dass die Couches während dem Verfahren der Liegen zwischen den Vorbereitungs-, Diagnostik- und Therapieräumen auf ihrer Unterseite über die Länge abgestützt werden müssen. Eine derartige Lösung ist beispielsweise in der US2007/0124858 A1 gezeigt. Dies wird insbesondere dann schwierig, wenn Liegen in dem Bereich, wo auch von der Unterseite bestrahlt werden kann, Ausnahmen haben und/oder verkürzt sind, um eine direkte Bestrahlung von der Unterseite zu ermöglichen.

Die Liegen haben eine Dicke respektive Höhe von ca. 5 cm. Dadurch verschiebt sich der Patient in der Höhenlage beim Gebrauch auf den Diagnostikmaschinen in vertikaler Richtung 5 cm nach oben. Wenn zusätzlich vorstehende Kupplungselemente an den Couches angebracht sind und auf die Diagnostikmaschinen die Gegenstücke der Kupplung aufgebaut sind, verschiebt sich der Patient um eine weitere Distanz (ca. 4 cm oder mehr) nach oben.

Dadurch kommen diejenigen Patienten mit einem etwas grösseren Umfang nicht mehr in den Bereich zu liegen, wo die Diagnostikmaschinen die qualitativ hochwertigen Bilder produzieren können.

Eine Aufgabe der Erfindung ist die Schaffung einer Transfereinrichtung, welche die Nachteile und Mängel der bekannten Lösungen behebt und mit welcher die Patienten auf der Therapieliege optimal platziert werden können.

Gelöst wird diese Aufgabe durch eine Transfereinrichtung für eine Therapieliege gemäss den Merkmalen des Patentanspruchs 1. Vorteilhafte Ausgestaltungen der Transfereinrichtung sind in den abhängigen Ansprüchen umschrieben.

Durch die Anordnung der Spannelemente innerhalb des flachen, nur wenige Zentimeter dicken Liegenkörpers wird es möglich, die Therapieliege unmittelbar auf den Therapietisch aufzulegen und damit die Aufbauhöhe an den Diagnostikmaschinen gegenüber den bekannten Liegen wesentlich zu verringern respektive auf die Couch-Stärke zu beschränken und gleichzeitig die Nutzung der Bildgebung der Diagnostikmaschinen wesentlich zu verbessern.

Damit diese Möglichkeiten ohne Einschränkungen gegeben sind, wird die Kupplung für die Liege zur Befestigung der Liege am Transportwagen an dem gegenüberliegenden Ende vom Therapiebereich der Liege angebracht, wodurch die Unterseite der Liege im Wesentlichen über deren gesamte Länge frei von störenden Metallteilen und frei und vollflächig für den Kontakt zu den Diagnostikmaschinen oder der Therapiemaschine bleibt. Wenn das Patientenpositioniersystem in die Unterseite der Liege eingreift, fährt das Patientenpositioniersystem etwas höher als notwendig. Dadurch wird erreicht, dass die Liege sauber aufliegt und auch, dass, falls die Kupplung in horizontaler Richtung nicht exakt positioniert ist, kein Kippen der Liege erfolgt. Die vertikale Bewegung wird durch das Positioniersystem ausgeführt, d. h. der Wagen, welcher die Liege trägt, weist deshalb lediglich eine vertikale Führung ohne Antrieb für die Kupplung auf. Nach der kurzen Vertikalbewegung kann die Liege von der Kupplung am Transportwagen gelöst werden. Unvermeidbare Höhendifferenzen zwischen der Liegenaufnahme am Transportwagen und den Oberflächen an den Tischen werden ausgeglichen. Die Übergabe von der Diagnostikmaschine an den Wagen erfolgt in umgekehrter Reihenfolge. Sobald die Liege vom Wagen übernommen wurde und das Patientenpositioniersystem sich abgesenkt hat, senkt sich auch die Liege etwas ab, bis sie auf einem mechanischen Anschlag an der Kupplung im Transportwagen aufliegt. Im nicht durchstrahlten Bereich der Liege können innerhalb der Liege Kupplungsteile angebracht sein, welche eine horizontale Justierung und Positionierung der Liege auf den Patiententischen an der Diagnostik und Therapie ermöglichen. Der Zentriermechanismus (passive Zentrierung) ist innerhalb der Liege eingebaut und orientiert sich an auf dem Patiententisch angebrachten Kupplungsteilen (aktive Zentrierung), welche beim Anheben des Patiententisches in die Liege hineinragen. Selbstverständlich können auch an der Liege (vorzugsweise innerhalb der Liegendicke) die aktiven Zentrierelemente und an den Tischen passive Zentrier- oder /und Spannelemente angeordnet sein.

Anhand eines illustrierten Ausführungsbeispiels wird die Erfindung näher erläutert. Es zeigen:

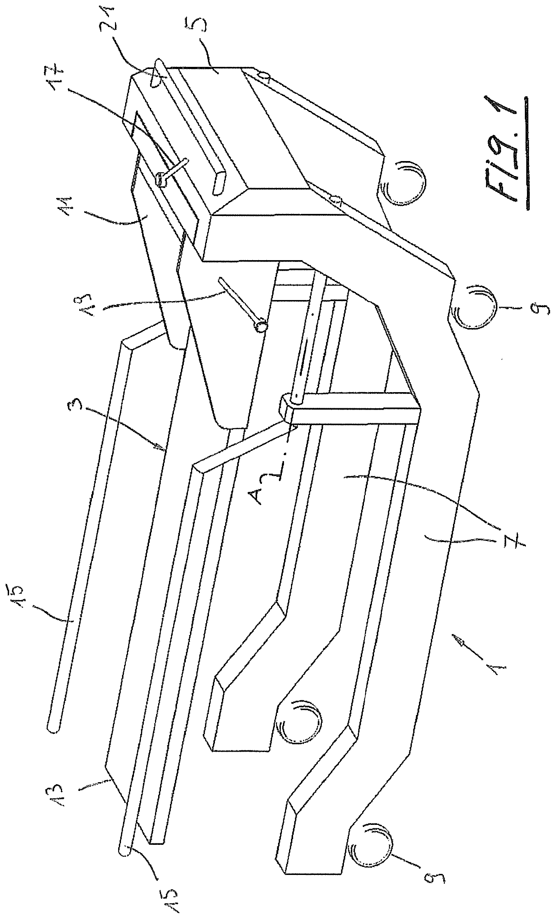

- Figur 1: einen Transportwagen mit einer angekuppelten Therapieliege,
- Figur 2: einen Transportwagen vor einer Diagnostikmaschine,
- Figur 3: einen Transportwagen an der Diagnostikmaschine kurz vor dem Aufsetzen der Therapieliege auf den Therapietisch,

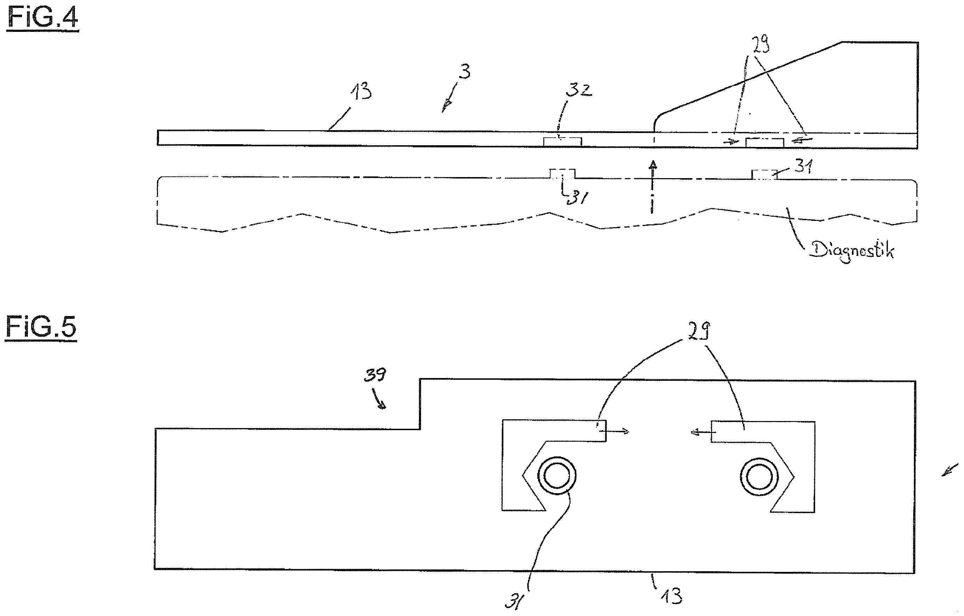

- Figur 4: einen Längsschnitt durch die Therapieliege, Figur eine Aufsicht auf die Therapieliege mit einem in den Liegenkörper integrierten Spannmittel,
- Figur 6: eine Seitenansicht der Therapieliege kurz vor dem Auflegen auf das Positioniersystem an der Therapiemaschine und
- Figur 7: eine Aufsicht auf die Therapieliege und das Positioniersystem, letzteres in gebrochenen Linien dargestellt.

Mit Bezugszeichen 1 ist ein Transportwagen für eine Therapieliege 3, auch Couch genannt, bezeichnet. Der Transportwagen 1 umfasst beispielsweise einen Kopfteil 5 mit einer Vertikalführung für eine Kupplungsvorrichtung. An der Unterkante des Kopfteils 5 sind zwei ein Fahrgestell bildende, seitlich beabstandet liegende Ausleger 7 mit je zwei Rädern 9 angeordnet sind. Im Kopfteil 5 sind die nicht angetriebene Vertikalführung und eine Kupplungsvorrichtung eingebaut (Vertikalführung und Kupplungsvorrichtung nicht dargestellt). Die Kupplungsvorrichtung dient dazu, einen Kupplungskopf 11, welcher mit der Therapieliege 3 bzw. den die Therapieliege 3 bildenden plattenförmigen Liegenkörpern 13 lösbar verbunden ist, zu tragen. Die Kupplungselemente am Kupplungskopf 11 für die lösbare Verbindung zur Therapieliege sind ebenfalls nicht dargestellt.

Am Transportwagen 1 können zudem zwei Haltestangen 15 befestigt sein, die um eine Achse A nach unten schwenkbar gelagert sind. Das Kuppeln der Therapieliege 3 an der Vertikalführung erfolgt über einen Hebel 17. Das Lösen der Verbindungskupplung zwischen der Vertikalführung im Kopfteil 5 und dem Kupplungskopf 11 erfolgt ebenfalls mit dem Hebel 17. Der Transportwagen 1 kann an einer Führungsstange 21 von Hand geschoben werden oder, falls erwünscht, sensorgesteuert durch einen elektrischen Antrieb der Räder 9 verfahren werden.

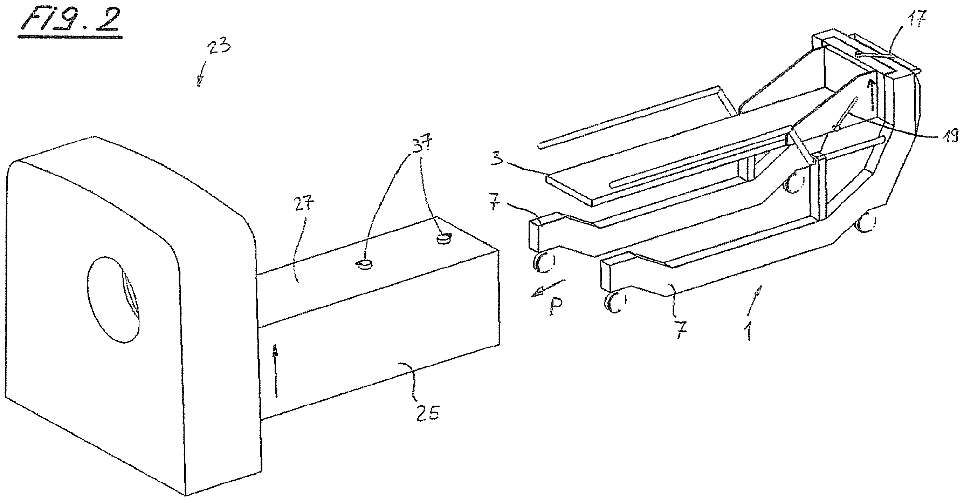

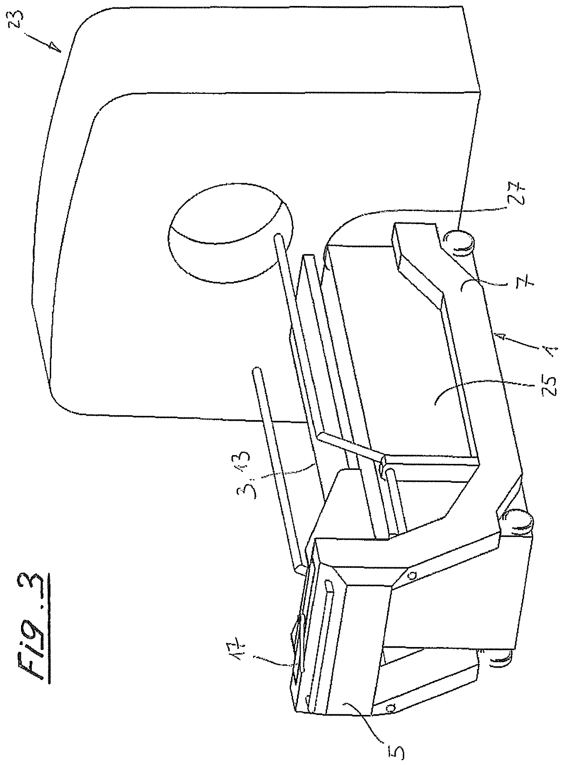

Der Abstand der beiden Ausleger 7 ist derart gewählt, dass diese beim Heranführen des Transportwagens 1 an eine Diagnostikmaschine 23 bzw. an deren Therapietisch 25 die Liege 3 über den Tisch 25 zu liegen kommen (Figuren 2 und 3). Wird der Transportwagen 1 in Richtung des Pfeils P (Figur 2) über den Therapietisch 25 geschoben, so liegt die Unterseite der Therapieliege 3 knapp über der Oberfläche 27 des Therapietischs 25. Ist die Endstellung etwa erreicht (Figur 3), hebt sich der Therapietisch 25. Durch die Hubbewegung wird die Therapieliege 3 leicht angehoben, was durch die Parallelführung im Kopfteil 5 möglich ist, ohne dass die Liege 3 vom Kopfteil 5 entkuppelt wird. Durch Betätigen des zweiten Hebels 19 wird die Therapieliege 3 auf der Oberfläche des Therapietisches 25 in horizontaler Lage zentriert. Der Zentriermechanismus 29 ist zwischen den beiden Oberflächen, d. h. der oben anliegenden Oberfläche und der untenliegenden Oberfläche, der Therapieliege in die Therapieliege 3 eingebaut und orientiert sich an auf dem Patiententisch 25 angebrachten Kupplungsteilen, z. Bsp. zylindrische Bolzen 31, welche mit dem Anheben des Therapietisches 25 von unten in einen Raum 32 an der Unterseite der Therapieliege 3 hineinragen. In der Unterseite bzw. im Inneren der Therapieliege 3 sind passive Kupplungsteile 35, z.B. hinterschnittene Bereiche ausgebildet, in welche aktive, d.h. aktiv bewegbare Kupplungsteile 37 eingreifen können und z.B. durch eine Spreizbewegung die passiven Kupplungsteile 35 hintergreifen. Durch die Spreizbewegung der aktiven Kupplungsteile 37, die am Therapietisch 25 angeordnet sind, erfolgt nicht nur das Festhalten der Therapieliege 3 auf dem Therapietisch 25, sondern vorab oder gleichzeitig eine absolut genaue reproduzierbare Positionierung der Therapieliege 3 in der horizontalen Ebene. Die beiden Kupplungsteile 35 und 37 bilden somit ein Positioniersystem 33 für die Therapieliege 3. Die passiven und aktiven Kupplungsteile 35,37 sind vorzugsweise aus nicht magnetischen Werkstoffen hergestellt, wodurch die Liege 3 beispielweise auch am MRI eingesetzt werden kann. Mit der erfindungsgemässen Ausbildung und Anordnung der Kupplungsteile 35,37 können an der Therapieliege 3 auch Ausschnitte 39 ausgebildet sein, welche eine optimale Zugänglichkeit der Bestrahlungsmaschine an einem auf der Therapieliege 3 liegenden Patienten ermöglichen. Nach dem Auflegen der Couch/Liege 3 auf den Tisch 25 an der Diagnostikmaschine oder dem Ankuppeln der Couch am Patientenpositioniersystem an der Therapiemaschine wird durch Betätigen des ersten Hebels 17 die Therapieliege 3 vom Kopfteil 5 des Transportwagens 1 getrennt. Der Transportwagen 1 kann nun vom Therapietisch 25 weggeführt werden.

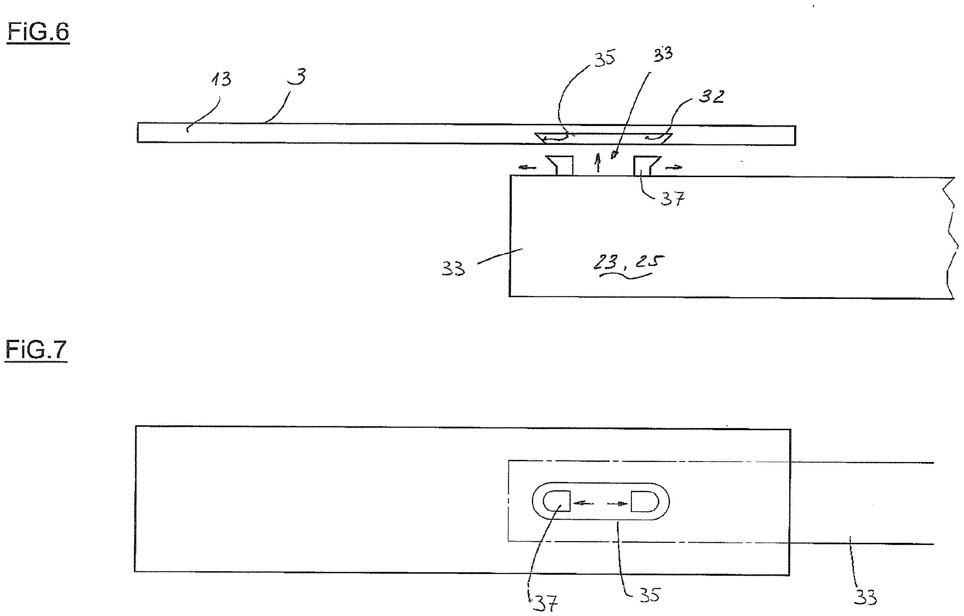

In der Ausgestaltung der Erfindung gemäss Figuren 6 und 7 ist der passive Kupplungsteil 35 im Liegenkörper 13 der Therapieliege 3 ausgebildet. In dieser Ausgestaltung ist der passive Kupplungsteil 35 ein Hinterschnitt im Raum 32, in welchem aktive Kupplungsteile 37, die am Tisch 23 der Diagnostikmaschine und am Tisch 25 der Therapiemaschine vertikal verschiebbar und horizontal spreizbar ausgebildet sind. Die Betätigung der aktiven Kupplungsteile an den Tischen 23,25 erfolgt durch elektromechanische oder handbetätigte, in den Figuren nicht dargestellte Aktivierungsmittel.

## Patentansprüche

1. System, umfassend eine Therapieliege (3) und eine Transfereinrichtung umfassend einen Transportwagen (1) mit einer Kupplungsvorrichtung für den Transfer der Therapieliege (3) von einem ersten Tisch an einer Diagnostikmaschine zu einem zweiten Tisch an einer Therapiemaschine zur Strahlenbehandlung einer auf der Therapieliege (3) liegenden Person, wobei die Therapieliege (3) einen flachen, plattenförmigen Liegenkörper (13) umfasst, zwischen dessen oben und unten anliegenden Oberflächen ein Raum (32) zum Einführen eines Kupplungsteils (29,37,31) von unten durch die untere Oberfläche des Liegenkörpers (13) und zum Positionieren und zum Festhalten der Therapieliege (3) auf den Tischen (23,25) ausgebildet ist, wobei zwischen den beiden Oberflächen ein Spannelement (29) vorhanden ist, mit dem die Therapieliege (3) an den Tischen der Diagnostik- und Therapiemaschine positionsgenau befestigbar ist, **dadurch gekennzeichnet, dass** die Therapieliege einen an dem gegenüberliegenden Ende vom Therapiebereich der Liege lösbar angebrachten Kopplungskopf aufweist, welcher mit der Kupplungsvorrichtung des Transportwagens verbindbar ist.

2. System nach Anspruch 1, **dadurch gekennzeichnet, dass** das Kupplungsteil als aktives Kupplungsteil (29, 37) ausgebildet ist.

3. System nach Anspruch 2, **dadurch gekennzeichnet, dass** der Raum (32) derart ausgebildet ist, dass dieser ein passives Kupplungsteil (35) bildet, in welches von unten das aktive Kupplungsteil (37) einfahrbar und zum Positionieren und Festhalten spreizbar ausgebildet ist.

4. System nach Anspruch 2, **dadurch gekennzeichnet, dass** der Raum (32) derart ausgebildet ist, dass in diesem das aktive Kupplungsteil (29) eingesetzt ist, welches an ein von unten einfahrbares passives Kupplungsteil (31) zum Positionieren und Festhalten angreift.

5. System nach einem der Ansprüche 1 bis 4, **dadurch gekennzeichnet, dass** die Therapieliege (3) einen Fussteil (5) umfasst, mit welchem die Therapieliege (3) kopfseitig am Transportwagen (1) festgehalten, getragen und zum Entkuppeln passiv vertikal bewegbar ist.

## Claims

1. A system comprising a stretcher (3) and a transfer device comprising a transport vehicle (1) with a coupling apparatus for transferring the stretcher (3) from a first table of a diagnosis machine to a second table for radiation treatment of a person lying on the stretcher (3), wherein the stretcher (3) comprises a flat, disc shaped stretcher body (13), a space (32) being formed between the upper and lower abutting surfaces thereof for inserting a coupling part (29, 37, 31) from below through the lower surface of the stretcher body (13) and for positioning and holding the stretcher (3) on the tables (23, 25), wherein a tensioning element (29) is present between the two surfaces with which the stretcher (3) is able to be positionally accurately fixed to the tables of the diagnosis and therapy machines,
**characterized in that** the stretcher has a coupling head detachably mounted at the end of the stretcher lying opposite the therapy region, said coupling head being connectible to the coupling apparatus of the transport vehicle.

2. The system according to Claim 1, **characterized in that** the coupling part is formed as an active coupling part (29, 37) .

3. The system according to Claim 2, **characterized in that** the space (32) is formed such that it shapes a passive coupling part (35) into which the active coupling part (37) is able to be introduced from below and which is expandably formed for positioning and holding.

4. The system according to Claim 2, **characterized in that** the space (32) is formed such that the active coupling part (29) is inserted therein, said coupling part engaging with a passive coupling part (31) being able to be introduced from below for positioning and holding.

5. The system according to any one of the Claims 1 to 4, **characterized in that** the stretcher (3) comprises a base part (5) with which the stretcher (3) is, at its head side, held on the transport vehicle (1), carried, and passively vertically movable for decoupling.

## Revendications

1. Système, comprenant un lit de traitement (3) et un moyen de transfert comprenant un chariot (1) avec un moyen d'accouplement pour le transfert du lit de traitement (3) depuis une première table sur une machine de diagnostic vers une seconde table sur une machine de traitement par radiothérapie d'une personne couchée sur le lit de traitement (3), dans lequel le lit de traitement (3) comprend un corps de lit (13) plat en forme de plateau, un espace (32) étant réalisé entre les surfaces du corps situées au-dessus et au-dessous pour introduire une partie d'accouplement (29,37,31) par le bas à travers la surface inférieure du corps de lit (13) et pour positionner et fixer le lit de traitement (3) sur les tables (23, 25), dans lequel un élément de serrage (29) est présent entre les deux surfaces et permet de fixer le lit de traitement (3) aux tables de la machine de diagnostic et de traitement dans la bonne position,
**caractérisé en ce que** le lit de traitement présente une tête d'accouplement appliquée de manière détachable sur l'extrémité opposée à la zone de traitement du lit et qui peut être reliée au moyen d'accouplement du chariot.

2. Système selon la revendication 1, **caractérisé en ce que** la partie d'accouplement est réalisée sous la forme d'une partie d'accouplement (29, 37) active.

3. Système selon la revendication 2, **caractérisé en ce que** l'espace (32) est réalisé de telle sorte qu'il forme une partie d'accouplement (35) passive, dans laquelle la partie d'accouplement (37) active peut être avancée par le bas et est réalisée déployable pour le positionnement et la fixation.

4. Système selon la revendication 2, **caractérisé en ce que** l'espace (32) est réalisé de telle sorte que la partie d'accouplement (29) active est insérée dans celui-ci et vient s'engrener dans une partie d'accouplement (31) passive avancée par le bas pour le positionnement et la fixation.

5. Système selon une des revendications 1 à 4, **caractérisé en ce que** le lit de traitement (3) comprend une partie de pied (5) qui permet de fixer le lit de traitement (3) au chariot (1) côté tête, de le porter et de le déplacer verticalement de manière passive pour le désaccouplement.
